(19) Europäisches Patentamt
European Patent Office
Office européen des brevets

(11) Publication number: **0 133 550**
**B1**

(12) # EUROPEAN PATENT SPECIFICATION

(45) Date of publication of patent specification: **18.10.89**

(51) Int. Cl.[4]: **C 12 P 13/00** // (C12P13/00, C12R1:01)

(21) Application number: **84109151.5**

(22) Date of filing: **02.08.84**

(54) **Process for producing arphamenine in high yield.**

(30) Priority: **05.08.83 JP 142527/83**

(43) Date of publication of application:
**27.02.85 Bulletin 85/09**

(45) Publication of the grant of the patent:
**18.10.89 Bulletin 89/42**

(84) Designated Contracting States:
**AT BE CH DE FR GB IT LI LU NL SE**

(56) References cited:
**EP-A-0 096 356**

**AGRICULTURAL AND BIOLOGICAL CHEMISTRY, vol. 48, no. 6, June 1984, pages 1661-1662, Tokyo, JP; SHOKICHI OHUCHI et al.: "The improvement of productivity and selective production of arphamenines by addition of their biosynthetic precursors**

(73) Proprietor: **MICROBIAL CHEMISTRY RESEARCH FOUNDATION**
**14-23, Kamiosaki 3 Chome Shinagawa-ku**
**Tokyo 141 (JP)**

(72) Inventor: **Umezawa, Hamao, Prof.**
**4-23, Toyotama-kita Nerima-ku**
**Tokyo (JP)**
Inventor: **Takeuchi, Tomio**
**5-1-11, Higashi-Gotanda Shinagawa-ku**
**Tokyo (JP)**
Inventor: **Aoyagi, Takaaki**
**3-3-6, Honkugenuma**
**Fujisawa-city Kanagawa Prefecture (JP)**

(74) Representative: **Becker, Heinrich Karl Engelbert, Dr. et al**
**HOECHST AKTIENGESELLSCHAFT Central Patent Department P.O. Box 80 03 20**
**D-6230 Frankfurt am Main 80 (DE)**

**EP 0 133 550 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to an improved process for producing arphamenine by fermentation.

It has been found that this substance is produced by the strain BMG361—CF4 of the *Chromobacterium violaceum* (FERM P—652). Its chemical structure is expressed by the following formula:

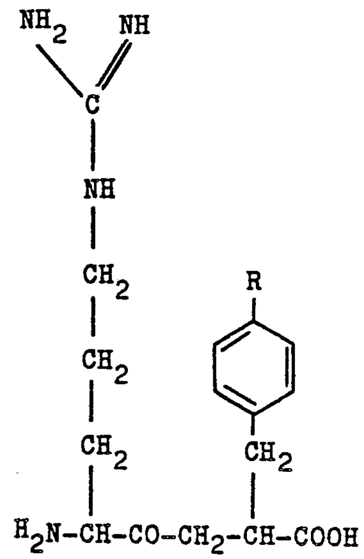

(wherein R is H or —OH)

Arphamenines expressed by the above formula are useful substances having low toxicity and showing antiaminopeptidase B activity, immunopotentiating action and various other pharmacological actions (see the specification of EP—A2—0 096 356).

Arphamenine is a generic name assigned to arphamenine A and arphamenine B. In the aforementioned general formula, arphamenine with a hydrogen atom as R refers to arphamenine A, and that with a hydroxy group as R, arphamenine B.

The specification of the above-cited European patent application discloses fermentation media preferred for the production of arphamenine. They include a medium comprising 1.5% glycerin, 1.5% soluble starch, 0.5% prorich, 1.5% fish meal and 0.2% calcium carbonate (weight/volume), and a medium comprising 3% soluble starch, 0.5% prorich, 1.2% corn gluten meal and 0.2% calcium carbonate (weight/volume). With these media, however, the productivity of arphamenine is never satisfactory for its commercial production.

We, the present inventors, therefore, have made extensive study on the composition of media in attempt to increase the yield of arphamenine on fermentation We have now found that the addition of at least one amino acid selected from L-arginine (as hydrochloride), L-phenylalanine and L-tyrosine in an amount of 0.2—2.0% by weight each to the culture medium unexpectedly increase the productivity of arphamenine to a marked level which is about 2 to 8 times higher than that attained by the use of the aforementioned media containing no such amino acids. The present invention has been accomplished on the basis of this finding. The present invention therefore relates to a process for producing arphamenine by an arphamenine-producing microorganism of the genus *Chromobacterium,* which is characterized in that at least one amino acid selected from L-phenylalanine, L-tyrosine and L-arginine is added singly or in combination to the culture medium.

Known nutrients for bacteria can be used, if desired, in culture medium for the production of arphamenine. Examples of carbon sources are fats and carbonhydrates, such as glycerin, glucose, lactose, sucrose, starch, maltose and molasses. Examples of nitrogen sources include peptone, meat extract, corn step liquor, cotton seed meal, peanut meal, soybean meal, corn gluten meal, fish meal, yeast extract, NZ-amine, casein, sodium nitrate, ammonium nitrate, ammonium sulfate, and ammonium phosphate. Examples of inorganic nutrients ae sodium chloride, phosphates, calcium carbonate and magnesium sulfate.

The effect of this invention will be described hereinafter by way of Experimental Examples.

The amount (potency) of arphamenine was determined by an improved method based on the method of Hoppusu et al. (V.K. HOPPUSU, K.K. MAKINEN & G.G. GLENNER, Archieves of Biochemistry and Biophysics *114,* 557, 1966). In detail, 0.5 ml of 0.1 M tris-hydrochloric acid buffer solution (pH 7.0) and 0.25 ml of a solution containing the specimen were added to 0.25 ml of 0.002 M arginine-ß-naphthylamide. The resulting mixed solution was heated for 3 minutes at 37°C. To the heated solution was added 5 µl of a solution of aminopeptidase B that had been purified by the use of DEAE-cellulose according to the enzyme-purification technique of Hoppusu et al.

The mixture was reacted for 30 minutes at 37°C, followed by adding to the reaction mixture 1 ml of 1.0

M acetic acid buffer solution (pH 4.2) containing Fast Garnet GBC® (0-aminoazotoluene diazonium salt) in a concentration of 1 mg/ml and the surfactant Tween 20® in a concentration of 10%.

The resulting mixture was allowed to stand for 15 minutes at room temperature, and measured for adsorbance (a) at 525 nm. Simultaneously, a blank absorbance (b) was obtained using an arphamenine-free buffer solution only. Percent inhibition of aminopeptidase B was calculated from the equation [(b-a)/b] × 100. According to this determination, the concentration of pure arphamenine A which inhibited 50% of aminopeptidase B activity, i.e., $IC_{50}$, was 0.005 μg/ml, and that of pure arphamenine B, 0.002 μg/ml. With these substances used as standards, the amount of arphamenine A or B was determined and its unit was expressed as potency by weight, such as in μg (potency) of mg (potency). The composition of culture media and the amounts of amino acids added were expressed as % (weight/volume).

The culture media of this invention and a conventional fermentation medium were compared in terms of the ability to produce arphamenine.

1. Composition of culture media

(1) Culture medium A of this invention
3% soluble starch, 0.5% prorich, 1.2% corn gluten meal, 0.2% calcium carbonate, 1.0% L-phenyllalanine, and the remainder being water.

(2) Culture medium B of this invention
3% soluble starch, 0.5% prorich, 1.2% corn gluten meal, 0.2% calcium carbonate, 1.0% L-phenylalanine, 0.5% L-artinine (monohydrochloride), and the remainder being water.

(3) Culture medium C of this invention
3% soluble starch, 0.5% prorich, 1.2% corn gluten meal, 0.2% calcium carbonate, 0.5% L-tyrosine, and the remainder being water.

(4) Conventional fermentation medium (control)
3% soluble starch, 0.5% prorich, 1.2% corn gluten meal, 0.2% calcium carbonate, and the remainder being water.

2. Strain used

*Chromobacterium violaceum* BMG361—CF4 (FERM—P No. 6521)

3. Conditions for cultivation

A 500 ml rotary flask was charged with 110 ml of the culture medium, and the medium was inoculated with 0.5 ml of cultured broth obtained by pre-cultivation. The inoculum was cultivated at 26—28°C at 180 rpm, and the potency of arphamenine was assayed with the passage of time.

The above-described cultured broth had been obtained in the following way: A primary culture medium comprising 3% soluble starch, 0.5% prorich, 1.2% corn gluten meal and 0.2% calcium carbonate (pH 7.4) was dispensed in amount of 110 ml into 500-ml rotary flasks. The dispensed medium was sterilized for 20 minutes at 120°C, and then inoculated with a loopful of the strain obtained by the slant-cultivation of *Chromobacterium violaceum* BMG361—CF4 (FERM P—6521). The inoculated medium was shake-cultured for 20 hours at 27°C to form cultured broth.

4. Results

The results are given in Table 1.

Table 1 Production of arphamenine

| Culture medium | Maximum potency | $IC_{50}$ (μl/ml) |
|---|---|---|
| Medium A of this invention | 190 μg/ml [(a)] (28-hour cultivation) | 0.026 |
| Medium B of this invention | 500 μg/ml [(a)] (28-hour cultivation) | 0.010 |
| Medium C of this invention | 170 μg/ml [(b)] (18-hour cultivation) | 0.012 |
| Conventional fermentation medium (control) | 40 μg/ml [(c)] (28-hour cultivation) | 0.088 |

Notes: (a): Calculated as arphamenine A (principal product) only.
(b): Calculated as arphamenine B (principal product) only.
(c): Calculated as arphamenine mixture (A : B = 1 : 1)

As clear from the above table, the medium A of this invention (main product: arphamenine A), the medium B of this invention (main product: arphamenine A), and the medium C of this invention (main product: arphamenine B) exhibited productivity of arphamenine A or B at 4—12 times as high as that of the conventional fermentation medium used as control.

Compared with the maximum potency of 70 μg/ml (calculated as a 1:1 mixture of arphamenine A and B from $IC_{50}$ of 0.05 μg.ml) obtained in the Example of the aforementioned European Patent Application, the arphamenine productivity of this invention is 2—7 times as high. This invention is noticeably characterized by its capability of predominantly producing arphamenine A or B.

Cultivation was performed in the same way as in Experiment Example 1 in order to determine the suitable ranges of the amounts of L-arginine, L-phenylalanine and L-tyrosine added. The composition of culture media were slightly modified, however, as described below.

1. Composition of culture media

A known fermentation medium consistiing of 3% soluble starch, 0.5% Prorich, 1.2% corn gluten meal, 0.2% calcium carbonate, and the remainder being water was used as the basal medium. To this basal medium were added varying amounts of L-arginine (hydrochloride), L-phenylalanine and L-tyrosine as shown in the above-mentioned table, to form various media. These media were used for cultivation.

2. Strain used

The same strain as in Experimental Example 1 was used.

3. Conditions for cultivation

The same conditions as in Experimental Example 1 were used.

4. Results

The results are shown in Table 2 or 3.

Table 2 Effect of the amounts of L-arginine and L-phenylalanine added on the production of arphamenine (maximum potency, μg/ml)

| | | L-phenylalanine (28-hour cultivation) | | | | | |
|---|---|---|---|---|---|---|---|
| | | 0 | 0.2 % | 0.5 % | 1.0 % | 1.5 % | 2.0 % |
| | 0 | 40[a] μg/ml | 160[b] | 170[b] | 190[b] | 560[b,c] | 500[b,e] |
| L-arginine (monohydro-chloride) | 0.5 % | 60[a] (0.060) | 160[b] (0.032) | 290[b] (0.017) | 500[b] (0.010) | 530[b,d] (0.0094) | 500[b,f] (0.010) |
| | 1.0 % | 30[a] (0.12) | 170[b] (0.029) | 310[b] (0.016) | 330[b] (0.015) | - | - |

Notes: (a): Calculated as a 1 : 1 mixture of arphamenine A and B.
(b): Calculated as arphamenine A only.
(c): 68-hour cultivation.
(d): 44-hour cultivation.
(e): 72-hour cultivation.
(f): 48-hour cultivation.
Values in parentheses represent $IC_{50}$ (μl/ml).

Table 3 Effect of the amount of L-tyrosine added on the production of arphamenine (maximum potency)

| 0 | 0.2 % | 0.5 % | 1.0 % |
|---|---|---|---|
| 40 (a) μg/ml | 130 (b) | 170 (b) | 130 (b) |
| (0.088) | (0.015) | (0.012) | (0.015) |

Notes: (a) Calculated as a 1 : 1 mixture of arphamenine A and B.

(b) Calculated as arphamenine B only.

Values in parentheses represent $IC_{50}$ (μl/ml).

The results in Table 2 show that the addition of 0.2—2% of L-phenylalanine alone or L-phenylalanine plus L-arginine (monohydrochloride) yields 160—560 μg/ml of arphamenine A, by far greater a potency than 40 μg/ml (potency) obtained when neither L-phenylalanine nor L-arginine was added. That is, said addition produced a 4- to 14-fold increase in arphamenine production over the level obtained when such addition was not peformed. Compared with the potency of 70 μg/ml shown in the Example of the aforementioned Japanese patent application, said addition brought about a 2- to 8-fold increase in arphamenine production.

The results in Table 3, on the other hand, show that the addition of 0.2—1.0% of L-tyrosine gave good increase in arphamenine production over the level obtained when this amino acid was not added.

These findings confirm that the addition of 0.2—2.0% of L-phenylalanine, the addition of 0.2—1.0% of L-tyrosine, or the addition of 0.5—1.0% of L-arginine (monohydrochloride) jointly with L-phenylalanine can specifically increase the production of arphamenine.

As described above, this invention relates to a process for producing arphamenine by cultivating an arphamenine-producing microorganism of the genus *Chromobacterium,* where at least one of amino acid selected from L-phenylalanine, L-tyrosine and L-arginine is added either singly or in combination in amounts of 0.2—2.0% to the culture medium, thereby affording the increased production of arphamenine compared with the use of conventional typical culture media. In this process, the addition of L-phenylalanine or L-phenylalanine plus L-arginine hydrochloride can produce arphamenine A principally, and the addition of L-tyrosine, arphamenine B principally.

## Example 3

800 liters of a culture medium consisting of 3.0% soluble starch, 0.5% Prorich, 0.2% calcium carbonate, 0.5% L-phenylalanine, 1.2% gluten meal, 0.3% monosodium phosphate, 0.05% antifoamer (KM—72®) in water was charged into a 2000-liter stainless steel tank, and sterilised for 20 minutes at 120°C. Then, the medium was inoculated with 16 liters of the cultured broth of *Chromobacterium violaceum* BMG361—CF4 (FERM—P No. 6521) that had been cultivated for 1 day with stirring under aeration, and the inoculated medium was cultivated for 45 hours at 28°C with stirring at 150 rpm under aeration with 800 liters of air/minutes. The filtrate of the resulting cultured broth showed 170 μg/ml (potency) as arphamenine A.

The cultured broth was adjusted to a pH of 2 with hydrochloric acid, and filtered to remove the mycelia and solid. The filtrate was adjusted to a pH of 6 with sodium hydroxide, and filtered again to obtain a 590-liter filtrate.

To the filtrate was added 5 kg of activated carbon (a product of Wako Pure Chemical, Japan, chromatography use), and the mixture was stirred for 1 hour. The activated carbon having arphamenine absorbed thereto was collected by filtration, and washed with 50 liters of water. The washed carbon was extracted 3 times with 40 liters each of 50% aqueous acetone at pH 2 adjusted with hydrochloric acid. The extract (108 liters) was adjusted to a pH of 5.0 wit sodium hydroxide, and concentrated under reduced pressure to obtain a 21.4-liter concentrate.

The concentrate was chromatographed on a 5-liter column of Amberlite XAD—4®, and the column was washed with 6 liters of water. The washed column was eluted sequentially with 6 liters of a 50% aqueous acetone and 4 liters of a 50% aqueous acetone (pH adjusted to 2.0 with HC1). Arphamenine appeared in the eluted in the latter half of the washing step with water. Thin-layer chromatography and high-performance liquid chromatography were performed to collect fractions containing arphamenine. These fractions were combined and concentrated under reduced pressure to obtain a 3.8-liter concentrate. The concentrate was adjusted to a pH of 4.5 with hydrochloric acid, and chromatographed on a 8-liter column of CM—Sephadex C—25® equilibrated with 0.05 M citric acid-sodium citrate under buffer solution (pH 4.5) containing 0.05 M NaCl. The column was pre-washed with 16 liters of the same buffer solution, and

subjected to a gradient elution between 20 liters of 0.05 M NaCl and 20 liters of 0.6 M NaCl in 0.05 M citric acid-sodium buffer solution.

The fractions containing arphamenine were combined, and concentrated under reduced pressure. Precipitates separated were removed from the concentrate by filtration, and the filtrate was chromatographed on a column containing 4-liter of CM—Sephadex C—25® equilibrated with the same buffer solution as described above. The column was pre-washed with 8 liters of 0.05 NaCl in the same buffer solution, and subjected to a gradient elution between 10 liters of 0.05 M NaCl and 10 liters of 0.5 M NaCl in the same buffer solution.

The fractions containing arphamenine were combined, and concentrated under reduced pressure. NaCl precipitated was removed by filtration, and the filtrate was chromatographed on a column containing 4-liter of Sephadex LH—20®. When the column was developed with water, it gave an arphamenine-containing fraction showing a single spot on thin-layer chromatography. The arphamenine-containing fraction was subjected to decolour with a small amount of activated carbon, and the decolourized material was freeze-dried to obtain 41.5 g of arphamenine A hydrochloride as power. The power showed an $IC_{50}$ of 0.005 μg/ml against aminopeptidase B.

Example 4

Fermentation was carried out in exactly the same way as in Example 1, except that L-tyrosine was used instead of L-phenylalanine in the culture medium. After 20 hours' fermentation, the filtrate of the cultured broth showed 179 μg/ml (potency) as arphamenine B.

The cultured broth was subjected to exactly the same filtration and purification steps as Example 1, and 22.0 g of arphamenine B hydrochloride was finally obtained as powder. This powder showed an $IC_{50}$ of 0.002 μg/ml against aminopeptidase B.

## Claims

1. A process for producing arphamenine in a high yield, by cultivating and arphamenine-producing microorganism belonging to the genus *Chromobacterium,* which comprises adding at least one of amino acid selected from L-phenylalanine, L-tyrosine and L-arginine singly or in combination to the culture medium.

2. A process as claimed in Claim (1) wherein the cultivation is performed with the addition of either L-phenylalanine alone or a mixture of L-phenylalanine and L-arginine to the culture medium, thereby predominantly producing arphamenine A.

3. A process as claimed in Claim (1) wherein the cultivation is performed with the addition of L-tyrosine to the culture medium, thereby predominantly producing arphamenine B.

4. A process as claimed in Claim (1) or (2) wherein L-phenylalanine or L-arginine is added in an amount of 0.2—2.0% to the culture medium.

5. A process as claimed in any of Claims (1) to (3) whereby L-tyrosine is added in an amount of 0.2—1.0% to the culture medium.

6. A process as claimed in any of Claims (1) to (5) whereby in the microorganism Chromobacterium Violaceum FERM P—6521 is used.

## Patentansprüche

1. Verfahren zur Herstellung von Arphamenin in hoher Ausbeute durch Kultivierung eines zur Gattung Chromobacterium gehörenden Arphamenin erzeugenden Mikroorganismus, dadurch gekennzeichnet, daß mindestens eine Aminosäure ausgewählt aus L-Phenylalanin, L-Tyrosin und L-Arginin allein oder Kombination zu dem Kulturmedium zugegeben wird.

2. Verfahren nach Anspruch 1, wobei die Kultivierung unter Zugabe von entweder L-Phenylalanin allein oder einer Mischung von L-Phenylalanin und L-Arginin zu dem Kulturmedium durchgeführt wird, damit vorwiegend Arphamenin A hergestellt wird.

3. Verfahren nach Anspruch 1, wobei die Kultivierung unter Zugabe von L-Tyrosin zu dem Kulturmedium durchgeführt wird, damit vorwiegend Arphamenin B hergestellt wird.

4. Verfahren nach Anspruch 1 oder 2, wobei L-Phenylalanin oder L-Arginin in einer Menge von 0,2 bis 2,0% zu dem Kulturmedium zugegeben wird.

5. Verfahren nach einem der Ansprüche 1 bis 3, wobei L-Tyrosin in einer Menge von 0,2 bis 1,0% zu dem Kulturmedium zugegeben wird.

6. Verfahren nach einem der Ansprüche 1 bis 5, wobei der Microorganismus Chromobacterium Violaceum FERM P—6521 verwendet wird.

## Revendications

1. Procédé de production d'arphaménine avec un rendement élevé par culture d'un microoganisme produisant de l'arphaménine et appartenent au genre *Chromobacterium,* qui comprend l'addition, au

milieu de culture, d'au moins un aminoacide choisi parmi la L-phénylalanine, la L-tyrosine et la L-arginine, seul ou en combinasion.

2. Procéde selon la revendication 1 dans lequel on effectue la culture en ajoutant au milieu de culture soit de la L-phénylalanine seule, soit un mélange de L-phénylalanine et de L-arginine, ce qui produit de façon prédominante de l'arphaménine A.

3. Procédé selon la revendication 1 dans lequel on effectue la culture en ajoutant au milieu de culture de la L-tyrosine, ce qui produit de façon prédominante de l'arphaménine B.

4. Procédé selon la revendication 1 ou 2 dans lequel on ajoute au milieu de culture de la L-phénylalanine ou de L-arginine en quantité de 0,2 à 2,0%.

5. Procédé selon l'une quelconque des revendications 1 à 3 dans lequel on ajoute au milieu de culture de la L-tyrosine en quantité de 0,2 à 1,0%.

6. Procédé selon l'une quelconque des revendications 1 à 5 dans lequel on utilise le microorganisme *Chromobacterium violacuem* FERM P—6521.